# DEMANDE DE BREVET EUROPEEN

(11) **EP 0 979 642 A1**
(43) Date de publication de la demande: **16.02.2000**
(21) Numéro de dépôt: 99401776.2
(22) Date de dépôt: 15.07.1999
(51) Int. Cl.: A61K 7/02

(54) **Composition de maquillage à haute viscosité comprenant une dispersion aqueuse de polymère**

(30) Priorité: 12.08.1998 FR 9810332
(71) Demandeur: L'OREAL, 75008 Paris (FR)
(72) Inventeur: Bara, Isabelle, 75013 Paris (FR); Jager-Lezer, Nathalie, 92340 Bourg-la-Reine (FR)
(74) Mandataire: Lhoste, Catherine

(57) **Abrégé**

L'invention a pour objet une composition pour le maquillage en relief comprenant une dispersion aqueuse de polymère filmogène, et un agent épaississant en une quantité efficace pour que la composition ait une viscosité, mesurée à 25 °C, à une vitesse de cisaillement de 200 s⁻¹, supérieure ou égale à 4,5 Pa.s (45 poises) et inférieure ou égale à 100 Pa. s (1000 poises).

L'invention a également pour objet un produit de maquillage en relief comprenant cette composition.

La composition selon l'invention permet notamment l'obtention de perles de maquillage.

## Description

La présente invention a pour objet une composition pour le maquillage en relief de la peau ou des phanères, contenant une dispersion aqueuse de polymère, l'utilisation d'une telle composition pour le maquillage en relief de la peau et des phanères, ainsi qu'un procédé de maquillage de ces derniers. La composition et le procédé de maquillage selon l'invention sont plus particulièrement destinés au maquillage de la peau du visage, y compris les lèvres, du corps, des paupières, des oreilles (et notamment le lobe), et des phanères tels que les cils, les sourcils, les cheveux et les ongles, d'êtres humains. L'invention porte aussi sur un produit de maquillage comprenant cette composition.

Les produits de maquillage sont couramment employés pour apporter de la couleur, mettre en valeur certaines parties de la peau ou des phanères, ou bien encore procurer un aspect brillant, mat, satiné à la peau ou aux phanères. Ces produits sont habituellement appliqués sous la forme d'une mince couche uniforme.

Pour conférer au produit de maquillage une bonne tenue sur la peau ou les phanères, il est connu d'utiliser des polymères filmogènes en dispersion aqueuse. Par exemple, le brevet US-A-3639572 décrit une composition de maquillage de la peau comprenant une dispersion aqueuse de polymère tels que les esters polyacryliques. Cette composition laisse après séchage un film de bonne tenue sur la peau. Le brevet US-A-4158053 décrit des vernis-à-ongles comprenant une dispersion aqueuse de polymère acrylique formant un film adhérant sur les ongles.

Avec l'évolution de la mode, les consommateurs, plus exigeants, recherchent de nouveaux produits de maquillage permettant d'obtenir des effets de maquillage originaux ou particuliers, et notamment conférant des transformations visibles du visage ou du corps maquillés.

Or, les compositions de maquillage actuellement disponibles sur le marché ne permettent que l'obtention d'un film uniforme et lisse et pas de maquillage en relief. Un besoin subsiste donc de disposer de produit de maquillage dont l'application sur la peau ou les phanères permettant d'obtenir un effet de maquillage différent des films uniformes et lisses actuellement obtenus avec les produits disponibles sur le marché.

La présente invention a donc pour but de proposer une composition de maquillage permettant d'obtenir un maquillage tridimensionnel, ou en relief, sur la peau et sur les phanères.

La demanderesse a constaté qu'un nouveau type de maquillage de la peau et/ou des phanères pouvait être obtenu à l'aide d'une composition comprenant une dispersion aqueuse de polymère et présentant une viscosité élevée particulière. La composition selon l'invention conduit ainsi à la formation d'un dépôt tridimensionnel sur la peau et/ou les phanères.

Le dépôt tridimensionnel est obtenu rapidement, voire instantanément, lors de l'application de la composition selon l'invention sur la peau et/ou sur les phanères. Ce dépôt tridimensionnel peut se présenter sous la forme de volumes sensiblement arrondis ou bombés, voire sensiblement sphériques ou hémisphériques. Après le dépôt de la composition, la surface du dépôt devient en particulier spontanément lisse et bombée, et les volumes déposés conservent leur forme après séchage complet de la composition. En particulier, le dépôt tridimensionnel peut se présenter sous la forme de perles (ou petites boules) ou bien encore sous la forme de galets.

Selon une autre variante de réalisation selon l'invention, il est possible de former avec la composition selon l'invention des cordons de maquillage ayant la forme d'un cylindre longitudinal, pouvant donner par exemple l'aspect d'un bracelet ou d'un collier.

De façon plus précise, la présente invention a pour objet une composition pour le maquillage en relief comprenant une dispersion aqueuse de polymère filmogène, et un agent épaississant en une quantité efficace pour que la composition ait une viscosité, mesurée à 25 °C, à une vitesse de cisaillement de 200 s⁻¹, supérieure ou égale à 4,5 Pa.s (45 poises) et inférieure ou égale à 100 Pa. s (1000 poises).

L'invention a également pour objet l'utilisation d'une composition telle que définie précédemment pour le maquillage en relief de la peau et/ou des phanères.

L'invention a aussi pour objet l'utilisation d'un agent épaississant, dans une composition pour le maquillage en relief comprenant une dispersion aqueuse de particules de polymère filmogène, en une quantité efficace pour que la composition ait une viscosité, mesurée à 25 °C, à une vitesse de cisaillement de 200 s⁻¹, supérieure ou égale à 4,5 Pa.s

L'invention a encore pour objet un procédé de maquillage en relief de la peau et/ou des phanères consistant à appliquer sur la peau et/ou les phanères une composition telle que définie précédemment.

L'invention a aussi pour objet un produit de maquillage en relief comprenant un réservoir contenant la composition de maquillage décrite précédemment, ledit réservoir étant en communication avec un orifice de distribution de ladite composition et comportant des moyens de pression permettant la distribution de la composition à travers l'orifice.

Dans la pratique, la composition selon l'invention a avantageusement une viscosité, mesurée à 25 °C et à une vitesse de cisaillement de 200 s⁻¹, inférieure ou égale à 100 Pa. s (1000 poises) pour être manipulable. Au delà de cette viscosité, la composition est trop rigide et ne permet pas d'obtenir le maquillage recherché. La viscosité est mesurée avec un viscosimètre RHEOMAT RM 180 (Brookfield) équipé d'un mobile n°4, la mesure étant effectuée après 10 minutes de rotation du mobile (temps au bout duquel on observe une stabilisation de la viscosité et de la vitesse de rotation du mobile).

De préférence, la composition selon l'invention a une viscosité supérieure ou égale à 8 Pa.s (80 poises), et mieux va de 10 à 45 Pa.s. (100 à 450 poises).

Avantageusement, la composition selon l'invention a un comportement rhéofluidifiant strict, c'est-à-dire que la composition se fluidifie (diminution de la viscosité) sous l'effet d'un cisaillement croissant, ce comportement étant réversible (augmentation de la viscosité lors de la diminution du cisaillement). En particulier, la composition à un seuil d'écoulement τ_{c} inférieur ou égale à 5 Pa, ce qui signifie que la contrainte à partir de laquelle le produit s'écoule est proche de 0.

Par ailleurs, la composition a une viscosité η₀, mesurée à 25 °C, au repos, c'est à dire à un très faible cisaillement d'environ 10⁻² s⁻¹ allant de 1 Pa.s à 10 ⁵ Pa.s (10 poises à 10 ⁶ poises) et de préférence de 100 Pa.s à 10 ⁴ Pa.s (1000 poises à 10 ⁵ poises). Cette viscosité est mesurée à 25 °C avec un rhéomètre à contrainte imposée RS 75 de la société HAAKE, équipé d'un mobile à géométrie cône/plan, le cône ayant un diamètre de 20 mm, un angle de 1° et un entrefer (distance entre le plateau sur lequel est déposée la composition et l'extrémité du cône) de 40 µm.

Avantageusement, la composition selon l'invention présente un comportement viscoélastique, notamment avec un caractère visqueux dominant aux faibles valeurs de la contrainte de cisaillement (c'est-à-dire sous faibles déformations). Ce comportement viscoélastique est caractérisé par une consistance G* allant de 100 Pa à 5000 Pa, de préférence de 500 Pa à 2000 Pa, et mieux de 500 Pa à 1000 Pa. En outre, la composition peut présenter une élasticité δ pouvant aller de 25 ° à 70 °, et mieux de 30 ° à 60 °. La consistance G* et l'élasticité δ sont déterminées en fonction d'une contrainte sinusoïdale ayant une fréquence de 1 Hz, mesurées à l'aide du rhéomètre à contrainte imposée RS 75 de la société HAAKE.

La consistance G* correspond au rapport de la contrainte imposée sur la déformation mesurée ; l'élasticité δ correspond à l'angle de déphasage de la contrainte par rapport à la déformation. Ces caractéristiques, y compris le seuil d'écoulement τ_{c}, sont notamment définies dans l'ouvrage "Initiation à la rhéologie", G. Couarraze et J.L. Grossiord, 2ème édition, 1991, Edition Lavoisier-Tec 1 Doc.

Préférentiellement, la composition présente une reprise en thixotropie totale ( ou dit à 100 %) allant de 5 minutes à 50 minutes, mieux de 1 minute à 10 minutes, et encore mieux de 1 minute à 7 minutes, ce qui permet d'obtenir des perles de maquillage lors de la distribution de la composition à l'aide d'un tube, notamment d'un tube à canule.

La reprise en thixotropie est déterminée à 25 °C avec le rhéomètre à contrainte imposée RS 75 de la société HAAKE selon la méthode suivante :
On mesure la viscosité η₁ d'un échantillon de la composition à un taux de cisaillement de
   1 s⁻¹ après 10 minutes ;
puis, on mesure la viscosité η₂ du même échantillon à un taux de cisaillement de 100 s⁻¹ après 10 minutes ;
puis on soumet toujours le même échantillon à un taux de cisaillement de 1 s⁻¹ pendant 1 heure en suivant l'évolution de la viscosité η' ; on détermine alors le temps au bout duquel la viscosité η' = η₁, ce temps correspondant à la reprise en thixotropie.

La composition selon l'invention contient un polymère filmogène se présentant sous la forme de particules en dispersion aqueuse pour permettre la formation du dépôt tridimensionnel sur la peau et/ou les phanères.

Dans la présente demande, on entend par "polymère filmogène", un polymère apte à former à lui seul ou en présence d'un agent auxiliaire de filmification, un film isolable.

Par polymère sous forme de particules en dispersion aqueuse, on entend une phase contenant de l'eau et éventuellement un composé soluble dans l'eau, dans laquelle est dispersé directement le polymère sous forme de particules.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats). Les polymères filmogènes de type radicalaires peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

On utilise de préférence des polymères filmogènes radicalaires anioniques, c'est-à-dire des monomères ayant au moins un monomère à groupement acide.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C₁-C₂₀, de préférence en C₁-C₈, des (méth)acrylates d'aryle, en particulier d'aryle en C₆-C₁₀, des (méth)acrylates d'hydroxyalkyle, en particulier d'hydroxyalkyle en C₂-C₆.
Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle.
Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.
Parmi les (méth)acrylates d'aryle, on peut citer l'acrylate de benzyle et l'acrylate de phényle.
Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C₂-C₁₂. Parmi les N-alkyl (méth)acrylamides, on peut citer le N-éthyl acrylamide, le N-t-butyl acrylamide et le N-t-octyl acrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle.

Comme monomères styrèniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme polymère filmogène acrylique utilisable selon l'invention, on peut citer ceux vendus sous les dénominations NEOCRYL XK-90®, NEOCRYL A-1070®, NEOCRYL A-1090®, NEOCRYL BT-62®, NEOCRYL A-1079®, NEOCRYL A-523® par la société ZENECA, DOW LATEX 432® par la société DOW CHEMICAL.

On peut ainsi citer, parmi les polycondensats utilisables comme polymère filmogène, les polyuréthannes anioniques, cationiques, non-ioniques ou amphotères, les polyuréthannes-acryliques, les poly-uréthannes-polyvinylpirrolidones, les polyester-polyuréthannes, les polyéther-polyuréthannes, les polyurées, les polyurée/polyuréthannes, et leurs mélanges.

Le polyuréthanne filmogène peut être, par exemple, un copolymère polyuréthanne, polyurée/uréthanne, ou polyurée, aliphatique, cycloaliphatique ou aromatique, comportant seule ou en mélange :
- au moins une séquence d'origine polyester aliphatique et/ou cycloaliphatique et/ou aromatique, et/ou,
- au moins une séquence siliconée, ramifiée ou non, par exemple polydiméthylsiloxane ou polyméthylphénylsiloxane, et/ou
- au moins une séquence comportant des groupes fluorés.

Les polyuréthannes filmogènes tels que définis dans l'invention peuvent être également obtenus à partir de polyesters, ramifiés ou non, ou d'alkydes comportant des hydrogènes mobiles que l'on modifie par réaction avec un diisocyanate et un composé organique bifonctionnel (par exemple dihydro, diamino ou hydroxyamino), comportant en plus soit un groupement acide carboxylique ou carboxylate, soit un groupement acide sulfonique ou sulfonate, soit encore un groupement amine tertiaire neutralisable ou un groupement ammonium quaternaire. Comme polyuréthanne filmogène utilisable selon l'invention on peut utiliser ceux commercialisés sous les dénominations NEOREZ R-981®, NEOREZ R-974® par la société ZENECA, les AVALURE UR-405®, SANCURE 875®, SANCURE 2060®, AVALURE UR-425®, SANCURE 861® par la société GOODRICH.

Parmi les polycondensats filmogènes, on peut également citer les polyesters, les polyesters amides, les polyesters à chaîne grasse, les polyamides, et les résines époxyesters.

Les polyesters peuvent être obtenus, de façon connue, par polycondensation d'acides dicarboxyliques avec des polyols, notamment des diols.
L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexa-nedicarboxylique, l'acide isophtalique, l'acide téréphtalique, l'acide 2,5-norborane dicarboxylique, l'acide diglycolique, l'acide thiodipropionique, l'acide 2,5-naphtalènedicarboxylique, l'acide 2,6-naphta-lènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi: l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement -SO₃M, avec M représentant un atome d'hydrogène, un ion ammonium NH₄⁺ ou un ion métallique, comme par exemple un ion Na⁺, Li⁺, K⁺, Mg²⁺, Ca²⁺, Cu²⁺, Fe²⁺, Fe³⁺. On peut utiliser notamment un monomère aromatique bifonctionnel comportant un tel groupement -SO₃M.

Le noyau aromatique du monomère aromatique bifonctionnel portant en outre un groupement -SO₃M tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement -SO₃M : l'acide sulfoisophtalique, l'acide sulfotéréphtalique, l'acide sulfophtalique, l'acide 4-sulfonaphtalène-2,7-dicarboxylique.
On préfère utiliser dans les compositions objet de l'invention des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique. De tels polymères sont vendus par exemple sous le nom de marque Eastman AQ par la société Eastman Chemical Products.

Les polymères d'origine naturelle, éventuellement modifiés, peuvent être choisis parmi la résine shellac, la gomme de sandaraque, les dammars, les élémis, les copals, les polymères cellulosiques insolubles dans l'eau, et leurs mélanges.

On peut encore citer les polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthannes, les polyurées, les polyesters, les polyesteramides et/ou les alkydes. Ces polymères sont généralement appelés polymères hybrides.

La dispersion comprenant un ou plusieurs polymères filmogènes peut être préparée par l'homme du métier sur base de ses connaissances générales.

La taille des particules de polymères en dispersion aqueuse peut aller de 10 à 500 nm, et est de préférence de 20 à 300 nm.

Le polymère en dispersion aqueuse peut être présent dans la composition selon l'invention en une teneur allant de 1 % à 50 % en poids, de préférence de 5 % à 40% en poids, de matière sèche de polymères filmogènes par rapport au poids total de la composition.

La composition selon l'invention peut comprendre un agent auxiliaire de filmification favorisant la formation d'un film avec les particules du polymère filmogène. Un tel agent de filmification peut être choisi parmi tous les composés connus de l'homme du métier comme étant susceptibles de remplir la fonction recherchée, et notamment être choisi parmi les agents plastifiants et les agents de coalescence.

Le milieu de la composition de l'invention est avantageusement un milieu aqueux contenant de l'eau. La teneur en eau dans la composition peut aller de 10 % à 98 % en poids, par rapport au poids total de la composition, et mieux de 30 % à 90 % en poids.

Pour conférer à la composition selon l'invention la viscosité requise pour obtenir le dépôt tridimensionnel, la composition doit contenir un agent épaississant permettant d'ajuster la viscosité voulue.

Parmi les agents épaississants utilisables selon l'invention, on peut citer :
- les épaississants cellulosiques hydrosoluble tels que l'hydroxyéthylcellulose, la méthylcellulose, l'hydroxypropylcellulose et la carboxyméthylcellulose. Parmi celles-ci, on peut citer notamment les gommes vendues sous la dénomination de "Cellosize QP 44001H" par la Société Amercol,
- la gomme de guar, notamment celles vendues sous la dénomination VIDOGUM GH 175 par la société UNIPECTINE et sous la dénomination JAGUAR C par la société MEYHALL,
- la gomme de guar quaternisée vendue sous la dénomination de "Jaguar C-13-S" par la Société Meyhall,
- les gommes de guar non-ioniques comprenant des groupements hydroxyalkyle en C₁-C₆. On peut mentionner à titre d'exemple, les groupements hydroxyméthyle, hydroxyéthyle, hydroxypropyle et hydroxybutyle. De telles gommes de guar sont notamment vendues sous les dénominations commerciales JAGUAR HP8, JAGUAR HP60 et JAGUAR HP120, JAGUAR DC 293 et JAGUAR HP 105 par la société MEYHALL, ou sous la dénomination GALACTASOL 4H4FD2 par la société AQUALON.
- les gommes de xanthane, de caroube, de scléroglucane, de gellane, de rhamsan, de karoya,
- les alginates, la maltodextrine, l'amidon et ses dérivés, l'acide hyaluronique et ses sels,
- les argiles, et notamment les montmorillonites, les hectorites, les laponites
- les acides polyacryliques réticulés tels que les "Carbopol" de la Société Goodrich,
- les polymères poly(métha)crylates de glycéryle vendus sous les dénominations de "Hispagel" ou "Lubragel" par les Sociétés Hispano Quimica ou Guardian,
- la polyvinylpyrrolidone,
- l'alcool polyvinylique,
- les polymères et les copolymères réticulés d'acrylamide, tels que ceux vendus sous les dénominations de "PAS 5161" ou "Bozepol C" par la Société Hoechst, de "Sepigel 305" par la Société Seppic, ou de "Salcare SC95" par la Société Allied Colloïd, ou encore
- les homopolymères réticulés de chlorure de méthacryloyloxyéthyltriméthylammonium vendus sous la dénomination de "Salcare SC95" par la Société Allied Colloïd.
- les polymères associatifs et notamment les polyuréthanes associatifs.

Selon l'invention, l'agent épaississant est de préférence choisi parmi les polyuréthanes associatifs.

Les polyuréthannes associatifs sont des copolymères séquencés non ioniques comportant dans la chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

En particulier, ces polymères comportent au moins deux chaînes lipophiles hydrocarbonées, ayant de C₆ à C₃₀ atomes de carbone, séparées par une séquence hydrophile, les chaînes hydrocarbonées peuvent être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polymères peuvent être séquencés sous forme de tribloc ou multibloc. Les séquences hydrophobes peuvent donc être à chaque extrémité de la chaîne (par exemple : copolymère tribloc à séquence centrale hydrophile) ou réparties à la fois aux extrémités et dans la chaîne (copolymère multiséquencé par exemple). Les polymères peuvent être également en greffons ou en étoile.

De préférence, les polymères sont des copolymères triblocs dont la séquence hydrophile est une chaîne polyoxyéthylénée comportant de 50 à 1 000 groupements oxyéthylénés. En général les polyuréthannes associatifs comportent une liaison uréthanne entre les séquences hydrophiles, d'où l'origine du nom.

Par extension figurent aussi parmi les polyuréthannes associatifs, des polymères dont les séquence hydrophiles sont liées par d'autres liaisons chimiques, autre que la liaison polyuréthane, aux séquences lipophiles.

A titre d'exemple, des polymères associatifs utilisables dans l'invention, on peut citer le polymère C₁₆-OE₁₂₀-C₁₆ vendu par la société HULS (sous le nom Sérad FX1100, molécule à fonction uréthanne et poids moléculaire moyen en poids de 1300), OE étant un motif oxyéthyléné. Comme polymère associatif, on peut aussi utiliser aussi le Rhéolate 205 à fonction urée vendu par la société RHEOX ou encore le Rhéolate 208 ou 204. Ces polyuréthannes associatifs sont vendus sous forme pure.

Le produit DW 1206B de chez RHOM & HAAS à chaîne alkyle en C₂₀ et à liaison uréthanne, vendu à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Sérad FX1010, le Sérad FX1035 et le Serad 1070 vendus par la société HULS, le Rhéolate 255, le Rhéolate 278 et le Rhéolate 244 vendus par la société RHEOX. On peut aussi utiliser le produit DW 1206F et le DW 1206J, ainsi que l'Acrysol RM 184 ou l'Acrysol 44 de la société RHOM & HAAS, ou bien encore le Borchigel LW 44 de la société BORCHERS.

Les polymères associatifs utilisables dans l'invention sont en particulier ceux décrits dans l'article de G. Fonnum, J. Bakke et Fk. Hansen - Colloid Polym. Sci 271, 380.389 (1993).

Dans la composition selon l'invention, l'agent épaississant est présent en une quantité efficace pour que la composition présente la viscosité telle que définie précédemment. La teneur en agent épaississant peut par exemple aller de 0,1 % à 20 % en poids, par rapport au poids total de la composition, et de préférence de 1 % à 10 % en poids.

Pour favoriser le séchage rapide de la composition après la formation du dépôt tridimensionnel sur la peau et/ou les phanères, la composition peut comprendre des accélérateurs de séchage comme les solvants volatils et de préférence des solvants organiques volatils miscibles à l'eau, comme l'éthanol. La quantité de tels solvants organiques est choisie de telle sorte que la viscosité de la composition soit bien maintenue dans la gamme définie précédemment. Ces solvants organiques peuvent être présent dans la composition en une teneur allant jusqu'à 15 % en poids, par rapport au poids total de la composition, (notamment de 0,1 % à 15 %) et de préférence jusqu'à 10 % en poids (notamment de 0,5 % à 10 %). Par volatil, on entend un composé apte à s'évaporer au contact de la peau, à température ambiante.

La composition peut en outre comprendre d'autres ingrédients habituellement utilisés en cosmétiques. De tels ingrédients peuvent être notamment des agents plastifiants, des agents de coalescence, des charges, des matières colorantes comme les pigments ou les colorants, des cires, des tensio-actifs, des conservateurs, des huiles, des agents hydratants, des parfums qui sont bien connus de l'état de la technique. Bien entendu, l'homme du métier veillera à choisir ce ou ces éventuels additifs et/ou leur quantité, de manière telle que les propriétés avantageuses dépôt tridimensionnel sur la peau et/ou les phanères soient conservées.

La composition peut être appliquée sur la peau et/ou les phanères à l'aide de tout applicateur connu de l'homme du métier tels que les brosses, les applicateurs à embout mousse, à plume floquée, en feutre, les pinceaux, les spatules. L'applicateur doit être choisi de telle sorte que le dépôt de la composition selon l'invention permette la formation de dépôt tridimensionnel comme décrit précédemment.

Un autre objet de l'invention est un produit de maquillage en relief comprenant d'une part la composition de maquillage en relief telle que décrite ci-dessus, et d'autre part un dispositif de conditionnement particulier de ladite composition.

Le dispositif de conditionnement pour la composition selon l'invention peut comprendre un réservoir, destiné à contenir la composition de maquillage, en communication avec un orifice de distribution de la composition et comportant des moyens de pression permettant la distribution de la composition à travers l'orifice. Selon un mode de réalisation du dispositif de conditionnement, le réservoir peut comporter une paroi souple constituant les moyens de pression permettant la distribution de la composition à travers l'orifice du réservoir. Un tel réservoir peut être notamment un tube à paroi souple.

En outre, le réservoir peut être muni d'un embout de distribution muni d'une extrémité libre comportant l'orifice de distribution de la composition. L'embout de distribution peut être notamment une canule. L'orifice de distribution a de préférence une section permettant la distribution de la composition sous forme de perles individuelles ou bien encore sous la forme d'un cordon.
Avantageusement, l'orifice de distribution peut avoir une section allant de 0,03 mm² à 80 mm².
Selon un variante de réalisation du produit selon l'invention, l'orifice de distribution peut être un orifice circulaire dont le diamètre peut aller de 0,1 mm à 5 mm.

Le dispositif de conditionnement décrit précédemment permet de déposer facilement la composition selon l'invention sur la peau (visage, paupières, lobe de l'oreille, doigts) ou sur les cheveux, les ongles, sous forme de perles individuelles, donnant ainsi un effet de maquillage en volume original. Ces perles peuvent avoir un diamètre allant de 1 mm à 6 mm.

La composition selon l'invention peut être également appliquée sur la peau ou les phanères à l'aide d'un pochoir: la composition ainsi déposée dans les parties évidées du pochoir conserve sa forme après le retrait du pochoir, donnant ainsi un maquillage sur la peau très décoratif.

L'invention est illustrée plus en détail dans les exemples suivants.

La description qui suit se réfère aux figures annexées dans lesquelles :

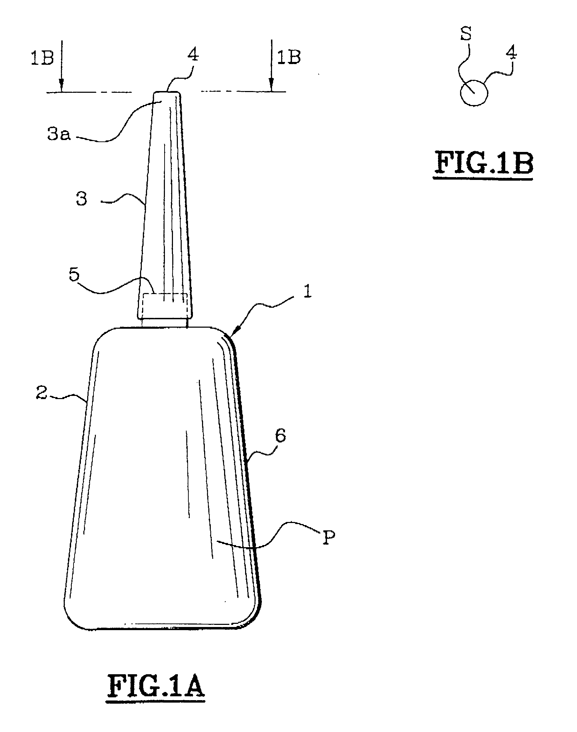

- la figure 1A représente, en coupe longitudinale, un produit pour le maquillage en relief conforme à l'invention,
- la figure 1B représente une coupe selon la ligne 1B-1B du dispositif de la figure 1A.

L'ensemble de conditionnement représenté à la figure 1A est désigné dans son ensemble par la référence 1. Il est constitué par un réservoir 2 en forme de tube renfermant la composition P à appliquer muni d'une ouverture 5. Un embout de distribution 3 en forme de canule est fixé sur l'ouverture 5 du réservoir 2. L'embout de distribution comporte à son extrémité libre 3a un orifice 4 de distribution de la composition P. Cet orifice 4 présente une section S circulaire, comme montré à la figure 1B, dont le diamètre peut par exemple aller de 0,1 mm à 5 mm.

Le tube 2 est muni d'une paroi 6 souple. Pour appliquer la composition avec le tube de la figure 1A, on exerce une pression sur la paroi 6 à l'aide des doigts pour que la composition contenue dans le tube soit expulsée du réservoir 2 à travers l'orifice de distribution, via la canule 3.
Ainsi, la paroi 6 souple du tube 2 constitue un moyen de pression permettant la distribution de la composition P à travers l'orifice 4 de distribution.
Avec ce dispositif de conditionnement, la composition P est distribuée sous forme de perles individuelles.

### Exemple 1:

On a préparé, par simple mélange à température ambiante, une composition de maquillage en relief ayant la composition suivante :
- dispersion aqueuse de polyuréthane (40 % de matières sèches) (SANCURE 861 de GOODRICH) 75 %
- polyuréthane associatif (Serad FX 1100 de SERVO HÜLS) 3 %
- pigment 5 %
- éthanol 7 %
- conservateurs qs
- eau qsp 100 %

La composition obtenue a une viscosité de 18,4 Pa.s (184 poises), mesurée à 25 °C, à l'aide du RHEOMAT RM 180, équipé du mobile n°4, à une vitesse de cisaillement de 200 s⁻¹, (mesure effectuée après 10 minutes de rotation).

La composition a également les caractérisdtiques rhéologiques suivantes, déterlminées selon les méthodes décrites aux exemples 3 à 8 ci-après :
- comportement rhéofluidifiant strict
- viscosité η₀ à 10⁻² s⁻¹ : 30000 Pa.s (3 10⁵ poises)
- consistance G*: 4 10³ Pa
- élasticité δ : 36 °
- reprise en thixotropie : 66 % en 5 minutes ; 95 % en 1 heure

La composition a été conditionnée dans un tube tel que décrit à la figure 1A.

La composition a été appliquée sous forme de perles sur l'extrémité des cils.

Cette même composition a également été appliquée sur les cheveux : on a déposé plusieurs perles de maquillage le long des mèches de cheveux, conférant un effet de maquillage très original.

La composition a aussi été déposée sur la peau : les billes formées adhèrent à la peau laissant sur celle-ci des perles de maquillage.

### Exemple 2 :

On a préparé, par simple mélange à température ambiante, une composition de maquillage ayant la composition suivante :
- dispersion aqueuse de polyuréthane (50 % de matières sèches) (SANCURE 2255 de GOODRICH) 81,6 %
- polyuréthane associatif (Borchigel LW 44 de BORCHERS) 1 %
- pigment 5 %
- conservateurs qs
- eau qsp 100 %

La composition obtenue a une viscosité de 5 Pa.s (50 poises) mesurée selon les conditions indiquées à l'exemple 1.

La composition a également les caractérisdtiques rhéologiques suivantes, déterlminées selon les méthodes décrites aux exemples 3 à 8 ci-après :
- comportement rhéofluidifiant strict
- viscosité η₀ à 10 ⁻² s⁻¹ : 100 Pa.s (1000 poises)
- consistance G*: 300 Pa
- élasticité δ : 60 °
- reprise en thixotropie totale à 1 minute

On a appliqué la composition sur la peau à l'aide d'un pochoir: après avoir retiré le pochoir, la composition appliquée garde sa forme et permet ainsi d'obtenir un maquillage décoratif sur la peau.

En outre, lorsque la composition est conditionnée dans un tube à canule comme celui décrit à la figure 1A, elle s'applique sur la peau sous forme de perles présentant l'aspect de galet.

### Exemples 3 à 8 : Etude comparative pour l'obtention de perles de maquillage

On a préparé 6 compositions (exemples 3 à 8) comprenant les ingrédients suivants, les teneurs étant exprimées en gramme :

| Exemple | 3 | 4 | 5 | 6 | 7 | 8 |
|---|---|---|---|---|---|---|
| Polyuréthane en dispersion aqueuse | 40 MA* | 31 MA** | 31 MA** | 31 g MA** | 47,7 g MA** | 39,3 g MA* |
| Agent épaississant | 2(*) | 0,5(*) | 0,7(*) | 1 (**) | 2,7(**) | 14,75 (***) |
| Ethanol | 3 | 3 | 3 | 3 | - | - |
| Conservateurs | qs | qs | qs | qs | qs | qs |
| Pigments | 5 | 5 | 5 | 5 | - | 5 |
| Eau | qsp 100 | qsp 100 | qsp 100 | qsp 100 | qsp 100 | qsp 100 |

| | | | | | | |
|---|---|---|---|---|---|---|
| * AVALURE UR-425 de la société GOODRICH | | | | | | |
| ** SANCURE 878 de la société GOODRICH | | | | | | |
| (*) Polyuréthane associatif SERAD FX 1100 de la société SERVO | | | | | | |
| (**) Hydroxyéthyl cellulose NATROSOL 250 M de la société HERCULES | | | | | | |
| (***) gel aqueux de laponite à 9 % en poids de laponite | | | | | | |

Pour chaque composition, on a mesuré la viscosité η₀ à 25 °C, avec le rhéomètre à contrainte imposée RS 75 de la société HAAKE équipé d'un cône/plan le cône ayant un diamètre de 20 mm, un angle de 1 ° et un entrefer de 40 µm. On a également mesuré la consistance G* et l'élasticité δ sous contrainte sinusoïdale à la fréquence de 1 Hz, ainsi que la reprise en thixotropie selon la méthode décrite précédemment.

Pour chaque composition, on a déterminé l'aptitude à former des perles lorsque la composition est conditionnée dans un tube muni d'une canule ayant un orifice circulaire d'un diamètre de 1 mm.

On a obtenu les résultats suivants :

| **Exemple** | **3** | **4** | **5** | **6** | **7** | **8** |
|---|---|---|---|---|---|---|
| Comportement | RS | RS | RS | RS | RP | RP |
| η₀ (Pa.s) | 380 | 400 | 3000 | 2 x 10 ⁴ | 2 x 10 ⁵ | 8 x 10 ⁵ |
| G* (Pa) | 600 | 900 | 1300 | 1700 | 29000 | - |
| δ (°) | 68 | 44 | 37 | 32,5 | 18 | - |
| Reprise en Thixotropie (minute) | 5 | 5 | 45 | 50 | 50 | - |
| Obtention de perles | sphérique | non sphérique | non sphérique | non sphérique | non | non |
| RS : rhéofluidifiant strict | | | | | | |
| RP : rhéofluidifiant plastique | | | | | | |
| η₀ : viscosité mesurée à 10 ⁻² s⁻¹ | | | | | | |

On constate que seules les compositions des exemples 3 à 6 permettent d'obtenir des perles distinctes, tandis que les compositions des exemples 7 et 8, dont la viscosité au repos η₀ est trop élevée, ne forment pas de perles mais un boudin.

## Revendications

1. Composition pour le maquillage en relief comprenant une dispersion aqueuse de polymère filmogène, caractérisée par le fait que la composition comporte un agent épaississant en une quantité efficace pour que la composition ait une viscosité, mesurée à 25 °C, à une vitesse de cisaillement de 200 s⁻¹, supérieure ou égale à 4,5 Pa.s et inférieure ou égale à 100 Pa. s.

2. Composition selon la revendication 1, caractérisée par le fait que la viscosité va de 10 Pa.s à 45 Pa.s.

3. Composition pour le maquillage en relief comprenant une dispersion aqueuse de polymère filmogène, caractérisée par le fait que la composition comporte un agent épaississant en une quantité efficace pour que la composition ait une viscosité η₀, mesurée à 25 °C, à un cisaillement de 10⁻² s⁻¹ allant de 1 Pa.s à 10 ⁵ Pa.s.

4. Composition selon la revendication 3, caractérisée par le fait que la viscosité va de 100 Pa.s à 10 ⁴ Pa.s.

5. Composition selon l'une quelconque des revendications précédentes, caractérisée par le fait qu'elle a une consistance G* allant de 100 Pa à 5000 Pa, de préférence de 500 Pa à 2000 Pa, et mieux de 500 Pa à 1000 Pa, mesurée sous une contrainte sinusoïdale ayant une fréquence de 1 Hz.

6. Composition selon l'une quelconque des revendications précédentes, caractérisée par le fait qu'elle a une élasticité δ pouvant aller de 25 ° à 70 °, et mieux de 30 ° à 60 °, mesurée sous une contrainte sinusoïdale ayant une fréquence de 1 Hz.

7. Composition selon l'une quelconque des revendications précédentes, caractérisée par le fait qu'elle présente une reprise en thixotropie totale allant de 5 minutes à 50 minutes, mieux de 1 minute à 10 minutes, et encore mieux de 1 minute à 7 minutes.

8. Composition selon l'une quelconque des revendications précédentes, caractérisée par le fait qu'elle a un comportement rhéofluidifiant strict.

9. Composition selon l'une quelconque des revendications précédentes, caractérisé par le fait que le polymère filmogène est choisi dans le groupe formé par les polymères radicalaires, les polycondensats, les polymères d'origine naturelle et leurs mélanges.

10. Composition selon la revendication 9, caractérisé par le fait que les polymères radicalaires sont choisis dans le groupe formé par les polymères vinyliques résultant de la polymérisation de monomères choisis parmi les acides carboxyliques insaturés α,β-éthyléniques, les esters de cesdits acides, les amides de cesdits acides, les esters vinyliques, les monomères styréniques.

11. Composition selon la revendication 10, caractérisé par le fait que les esters des acides carboxyliques insaturés α,β-éthyléniques sont choisis parmi les (méth)acrylates.

12. Composition selon la revendication 9, caractérisé par le fait que les polycondensats sont choisis dans le groupe formé par les polyuréthanes, les polyesters, les polesters amides, les polyamides, les résines époxyesters.

13. Composition selon la revendication 9, caractérisé par le fait que les polymères d'origine naturelle sont choisis dans le groupe formé par la résine shellac, la gomme de sandaraque, les dammars, les élémis, les copals, les polymères cellulosiques insolubles dans l'eau, et leurs mélanges.

14. Composition selon l'une quelconque des revendications précédentes, caractérisé par le fait que l'agent épaississant est choisi dans le groupe formé par les épaississants cellulosiques, les gommes de guar, les gommes de xanthane, de caroube, de scléroglucane, de gellane, de rhamsan, de karoya, les alginates, la maltodextrine, l'amidon et ses dérivés, l'acide hyaluronique et ses sels, les argiles, les acides polyacryliques réticulés, les poly(métha)crylates de glycéryle, la polyvinylpyrrolidone, l'alcool polyvinylique, les polymères et les copolymères réticulés d'acrylamide, les homopolymères réticulés de chlorure de méthacryloyloxyéthyltriméthylammonium, les polymères associatifs.

15. Composition selon l'une quelconque des revendications précédentes, caractérisé par le fait que l'agent épaississant est choisi parmi les polyuréthanes associatifs.

16. Composition selon l'une quelconque des revendications précédentes, caractérisée par le fait qu'elle comprend en outre au moins un ingrédient choisi dans le groupe formé par les agents plastifiants, les agents de coalescence, les charges, les matières colorantes, les cires, les tensio-actifs, les conservateurs, les huiles, les agents hydratants, les parfums.

17. Utilisation d'une composition telle que définie selon l'une quelconque des revendications précédentes, pour le maquillage en relief de la peau et/ou des phanères, le maquillage se présentant sous la forme d'un dépôt tridimensionnel déposé sur la peau et/ou les phanères.

18. Utilisation selon la revendication 17, caractérisé par le fait que le dépôt tridimensionnel se présente sous la forme de perles.

19. Utilisation selon la revendication 18, caractérisée par le fait que les perles ont un diamètre allant de 1 mm à 6 mm.

20. Utilisation d'un agent épaississant, dans une composition pour le maquillage en relief comprenant une dispersion aqueuse de particules de polymère filmogène, en une quantité efficace pour que la composition ait une viscosité, mesurée à 25 °C, à une vitesse de cisaillement de 200 s⁻¹, supérieure ou égale à 4,5 Pa.s.

21. Utilisation d'un agent épaississant, dans une composition pour le maquillage en relief comprenant une dispersion aqueuse de particules de polymère filmogène, en une quantité efficace pour que la composition ait une viscosité η₀, mesurée à 25 °C, à un cisaillement de 10⁻² s⁻¹ allant de 1 Pa.s à 10 ⁵ Pa.s (10 poises à 10 ⁶ poises).

22. Procédé de maquillage de la peau et/ou des phanères d'être humain, consistant à appliquer sur la peau et/ou les phanères une composition telle que définie selon l'une quelconque des revendications 1 à 16.

23. Procédé de maquillage de la peau selon la revendication 22, caractérisée par le fait que la composition est appliquée sur la peau à l'aide d'un pochoir.

24. Produit de maquillage en relief (1) comprenant un réservoir (2) contenant une composition de maquillage (P) selon l'une quelconque des revendications 1 à 16, ledit réservoir étant en communication avec un orifice (4) de distribution de ladite composition et comportant des moyens de pression (6) permettant la distribution de la composition à travers l'orifice (4).

25. Produit selon la revendication 24, caractérisé par le fait que l'orifice est situé à l'extrémité libre d'un embout de distribution (3).

26. Produit selon l'une quelconque des revendications 24 ou 25, caractérisé par le fait que le réservoir comporte une paroi souple (6) constituant les moyens de pression permettant la distribution de la composition à travers l'orifice.

27. Produit Selon l'une quelconque des revendications 24 à 26, caractérisé par le fait que le réservoir est un tube à paroi souple.

28. Produit selon l'une quelconque des revendications 24 à 27, caractérisé par le fait que l'orifice de distribution a une section allant de 0,03 mm² à 80 mm².
